# FASCICULE DE BREVET EUROPEEN

(11) **EP 2 222 633 B1**
(45) Date de publication et mention de la délivrance du brevet: **17.04.2013**
(21) Numéro de dépôt: 08854361.6
(22) Date de dépôt: 25.11.2008
(51) Int. Cl.: C07C 313/04

(54) **PROCEDE DE PREPARATION D'UN SEL DE L'ACIDE TRIFLUOROMETHANESULFINIQUES.**
VERFAHREN ZUR HERSTELLUNG EINES TRIFLUORMETHANSULPHINSÄURESALZES
METHOD FOR PREPARING A TRIFLUOROMETHANESULPHINIC ACID SALT.

(30) Priorité: 27.11.2007 FR 0708282
(43) Date de publication de la demande: 01.09.2010
(73) Titulaire: Rhodia Opérations, 93306 Aubervilliers (FR)
(72) Inventeur: SCHANEN, Vincent, F-69006 Lyon (FR); BUISINE, Olivier, F-69230 Saint Genis Laval (FR); METZ, Bernard, F-69540 Irigny (FR); BESSON, Bernard, F-01700 Les Echets (FR)
(74) Mandataire: Dutruc-Rosset, Marie-Claude
(86) Numéro de dépôt international: PCT/EP2008/066162
(87) Numéro de publication internationale: WO 2009/068534

(56) Documents cités:
- EP-A- 0 735 023
- WO-A-01/49659
- US-A1- 2001 031 891

## Description

La présente invention a pour objet un procédé de préparation d'un sel de l'acide trifluorométhanesulfinique.

L'invention vise la préparation d'un sel d'acide trifluorométhanesulfinique de haute pureté et plus particulièrement un sel alcalin de l'acide trifluorométhanesulfinique.

L'acide trifluorométhanesulfinique dénommé couramment « acide triflinique » ou bien ses formes salifiées sont des produits utilisés dans de nombreux domaines, phytosanitaire, pharmaceutique ou autre.

L'une des voies de synthèse dudit acide décrite dans EP 0 735 023, consiste à faire réagir l'acide trifluoroacétique, au moins partiellement salifié par un cation organique ou minéral avec du dioxyde de soufre dans un solvant organique polaire et chauffage du mélange résultant à une température comprise entre 100°C et 200°C pendant une durée comprise entre 30 min et 20 heures.

Les quantités relatives de l'acide trifluoroacétique et du dioxyde de soufre sont telles que le rapport entre le nombre d'atomes de soufre par mole d'acide trifluoroacétique est compris entre 1 et 10, avantageusement aux alentours de deux.

En fin de réaction, on obtient l'acide trifluoroacétique, l'acide triflinique sous forme saline, de préférence sous forme d'un sel de métal alcalin, préférentiellement le sodium ou le potassium et le solvant organique.

Sont également coproduits au cours de la réaction des sels, fluorure ou sulfate, le plus souvent sous forme de sel d'un métal alcalin, de préférence le sodium ou le potassium. Ces sels formés sont dénommés « impuretés salines » dans la suite du présent texte. Il est déjà connu de procéder au traitement d'un effluent de ce type contenant du trifluorométhanesulfinate de potassium, du trifluoroacétate de potassium et du diméthylformamide pour récupérer un solide essentiellement à base de trifluorométhanesulfinate de potassium (WO 2001/49659).

A l'issue de la réaction en présence de dioxyde de soufre décrite ci-dessus, on effectue une dilution par addition d'eau puis l'on extrait le solvant organique polaire par un solvant organique approprié, par exemple un hydrocarbure aliphatique chloré.

On sépare les phases organique et aqueuse,

On obtient généralement une solution aqueuse ayant une teneur en matières sèches de 10 à 40 % en masse comprenant :
- de 5 à 35 % en masse d'un sel d'acide triflinique, de préférence un sel alcalin,
- de 5 à 35 % en masse d'un sel d'acide trifluoroacétique, de préférence un sel alcalin,
- de 0,5 à 2 % en masse d'impuretés salines.

Ladite solution aqueuse comprend préférentiellement de 15 à 20 % en masse d'un sel d'acide triflinique, de 10 à 15 % en masse d'un sel d'acide trifluoroacétique et de 0,5 à 2 % en masse d'impuretés salines.

L'objectif de la présente invention est de fournir un sel d'acide triflinique de haute pureté à partir d'une phase aqueuse comprenant un sel d'acide triflinique associé à un sel d'acide trifluoroacétique et des impuretés salines résultant de son procédé de préparation.

Par « haute pureté », on entend dans le présent texte, un sel d'acide triflinique ayant une pureté supérieure ou égale à 90 %, de préférence supérieure ou égale à 95 %.

Ainsi, la présente invention a pour objet un procédé de préparation d'un sel de l'acide triflinique de haute pureté, à partir d'un mélange aqueux le comprenant associé à un sel d'acide trifluoroacétique et des impuretés salines résultant de son procédé de préparation caractérisé par le fait que ledit mélange est soumis aux opérations suivantes :
- première acidification contrôlée de telle sorte que l'on libère essentiellement le sel de l'acide trifluoroacétique sous sa forme acide, la majorité de l'acide triflinique restant sous une forme salifiée,
- séparation éventuelle des sels provenant de l'acide ayant servi à l'acidification et récupération d'une phase aqueuse,
- séparation de l'acide trifluoroacétique à partir de la phase aqueuse séparée comprenant le sel alcalin de l'acide triflinique, l'acide trifluoroacétique, l'acide triflinique, l'acide fort en excès, conduisant alors à une phase aqueuse épuisée en acide trifluoroacétique mais comprenant le sel alcalin de l'acide triflinique,
- récupération du sel alcalin de l'acide triflinique à partir de la phase aqueuse.

Selon un premier mode de réalisation de l'invention, la séparation de l'acide trifluoroacétique concomitamment avec l'acide triflinique peut se faire par traitement de la phase aqueuse séparée comprenant le sel alcalin de l'acide triflinique, l'acide trifluoroacétique, l'acide triflinique, l'acide fort en excès à l'aide d'un solvant organique susceptible d'extraire les acides organiques formés (acide trifluoroacétique, acide triflinique) suivi par la séparation des phases organique et aqueuse.

Selon une autre variante du procédé de l'invention, l'acide trifluoroacétique est séparée à partir de la phase aqueuse comprenant le sel alcalin de l'acide triflinique, l'acide trifluoroacétique, l'acide triflinique, l'acide fort en excès, par distillation.

Le procédé de l'invention est fondé sur le fait que l'on effectue une séparation du sel de l'acide trifluoroacétique du sel de l'acide triflinique en effectuant une acidification contrôlée du mélange aqueux les comprenant de telle sorte que l'on transforme essentiellement le sel de l'acide trifluoroacétique sous une forme acide, la majorité de l'acide triflinique restant sous une forme salifiée.

Conformément au procédé de l'invention, on effectue dans une première étape, l'acidification contrôlée du mélange aqueux de départ.

L'acidification est réalisée à l'aide d'un acide fort ayant un pkₐ inférieur ou égal à 1.

Le pKa est défini comme la constante de dissociation ionique du couple acide/base, lorsque l'eau est utilisée comme solvant.

On choisit un acide fort qui avantageusement ne présente pas de caractère oxydant. Ainsi, l'acide nitrique n'est pas préféré. On fait appel plus préférentiellement à l'acide sulfurique, chlorhydrique ou phosphorique.

L'acide sulfurique est choisi préférentiellement.

La quantité d'acide fort mis en oeuvre est telle que le rapport entre le nombre de moles d'acide exprimé en ions H⁺ et le nombre de moles du sel d'acide trifluoroacétique varie entre 1 et 3, de préférence entre 1 et 2.

On fait appel avantageusement à une solution d'acide fort concentrée.

On utilise plus particulièrement les formes commerciales d'acides.

On peut citer notamment les solutions d'acide sulfurique à 95 ou 98 % en masse, d'acide chlorhydrique à 37 % en masse, d'acide phosphorique à 95 - 100 % en masse.

On peut également utiliser l'acide chlorhydrique sous forme gazeuse ou des oléums qui correspondent à l'acide sulfurique chargé d'anhydride sulfurique SO₃ dont la concentration peut varier entre 10 % et 60 % en masse. Des oléums à 20 %, 40 % ou 60 % en masse de SO₃ sont commercialement disponibles.

L'opération d'acidification est conduite avantageusement à une température comprise entre 0°C et 60°C, de préférence entre 0°C et 10°C.

En fin de traitement acide, on recueille une solution ou une suspension aqueuse comprenant le sel de l'acide triflinique, l'acide trifluoroacétique, l'acide triflinique, l'acide fort en excès, les sels formés suite à l'acidification et les formes acides correspondant aux impuretés salines.

Généralement cette suspension comprend de 10 à 20 % en masse du sel de l'acide triflinique, de 5 à 20 % en masse d'acide trifluoroacétique, de 30 à 80 % d'eau.

Selon une variante de l'invention, il est possible d'ajouter dans la solution ou la suspension aqueuse obtenue, un composé susceptible de piéger l'acide fluorhydrique, par exemple l'acide borique ou de la silice, par exemple à une teneur de 1 % en masse.

Avant de procéder à l'extraction des acides organiques, il est possible mais non impératif de procéder à une séparation des solides présents dans la suspension aqueuse (sels, silice etc...) selon les techniques classiques de séparation solide/liquide, de préférence par filtration.

Selon une première variante du procédé de l'invention, on sépare l'acide trifluoroacétique concomitamment avec l'acide triflinique par extraction liquide liquide à l'aide d'un solvant organique.

A cet effet, on procède à un traitement de la phase aqueuse séparée comprenant le sel alcalin de l'acide triflinique, l'acide trifluoroacétique, l'acide triflinique, l'acide fort en excès obtenue à l'aide d'un solvant organique présentant un atome porteur d'un doublet électronique libre afin d'extraire dans une phase organique, les acides organiques formés (acide trifluoroacétique, acide triflinique).

Comme exemples de tels solvants, on peut citer les solvants de type amine ou éther.

Des exemples préférés de solvants de type amine sont les amines ayant de 2 à 20 atomes de carbone et l'on peut mentionner tout particulièrement les amines primaires telles que l'éthylamine, la propylamine, l'isopropylamine : les amines tertiaires telles que la triéthylamine, la tris(dioxa-3,6-heptyl)amine commercialisée sous la dénomination TDA-1.

Pour ce qui est des solvants de type éther convenant au procédé de l'invention, on peut mentionner notamment les éthers aliphatiques, cycloaliphatiques ou aromatiques et, plus particulièrement, le méthyltertiobutyléther, l'oxyde de dipentyle, l'oxyde de diisopentyle, le diméthyléther de l'éthylèneglycol (ou 1,2-diméthoxyéthane), le diméthyléther du diéthylèneglycol (ou 1,5-diméthoxy-3-oxapentane), l'anisole, le vératrole ou les éthers cycliques, par exemple, le dioxane, le tétrahydrofurane.

Les solvants préférés sont le méthyltertiobutyléther, le diisopropyléther.

La quantité de solvant mis en oeuvre représente le plus souvent de 100 % à 1000 % du poids de la phase aqueuse, de préférence de 100 % à 500 %.

En fin d'extraction, on obtient deux phases : une phase aqueuse comprenant le sel de l'acide triflinique et éventuellement des sels formés suite à l'acidification (par exemple KHSO₄ lorsque l'acide sulfurique est mis en oeuvre pour l'acidification) et une phase organique comprenant le solvant organique, les acides organiques formés et éventuellement l'excès de l'acide utilisé à l'acidification.

Dans une étape suivante, on sépare les phases aqueuse et organique, selon les techniques classiques, décantation ou centrifugation et l'on récupère le sel alcalin de l'acide triflinique à partir de la phase aqueuse séparée.

Selon une deuxième variante du procédé de l'invention, on sépare l'acide trifluoroacétique à partir de la phase aqueuse séparée, par distillation.

On introduit la phase aqueuse précitée comprenant le sel de l'acide triflinique, l'acide trifluoroacétique, l'acide triflinique, l'acide fort en excès, les sels formés suite à l'acidification et les formes acides correspondant aux impuretés salines dans une colonne de distillation, et l'on élimine en tête de distillation, l'acide trifluoroacétique et éventuellement de l'eau et l'on récupère en pied de distillation le sel de l'acide triflinique accompagné de l'acide fort en excès, des sels des acides forts notamment ceux provenant de l'acidification et de l'eau.

La distillation est conduite à une température dans le bouilleur comprise entre 60°C et 90°C sous une pression allant de 700 mbar à 10 mbar.

Elle est effectuée dans un appareil de distillation classique.

L'homme du métier est parfaitement en mesure de choisir les moyens à mettre en oeuvre en fonction des composés à séparer.

On rappellera simplement ce qui suit. La taille (notamment le diamètre) des colonnes de distillation dépend du flux circulant et de la pression interne. Leur dimensionnement se fera donc principalement suivant le débit de mélange à traiter. Le paramètre interne qu'est le nombre d'étages théoriques est déterminé notamment par la pureté du composé de départ et la pureté du produit devant être obtenus en tête de distillation.

On précisera que les colonnes pourront être garnies indifféremment de plateaux ou de garnissage ordonné, comme cela est parfaitement connu de l'homme du métier.

L'installation étant déterminée, l'homme du métier ajuste les paramètres de fonctionnement de la colonne.

Ainsi, la colonne de distillation pourra être avantageusement, mais non limitativement, une colonne ayant les spécifications suivantes :
- nombre d'étages théoriques : de 1 à 10, de préférence de 1 à 5,
- taux de reflux R compris entre 1 et 20 , de préférence entre 5 et 10.

En bas de colonne, on récupère un culot de distillation comprenant le sel de l'acide triflinique et en tête de colonne une phase gazeuse constituée par l'acide trifluoroacétique accompagnée éventuellement d'eau.

On refroidit la phase gazeuse et la transforme sous forme liquide par refroidissement à une température par exemple comprise entre -20°C et 20°C, de préférence comprise entre -10°C et 10°C.

Cette opération est conduite par passage dans un condenseur qui est un appareil classique par exemple un échangeur tubulaire alimenté par de l'eau ou par un fluide maintenu à une température voisine de la température de refroidissement choisie.

On choisit avantageusement pour la conduite de l'opération de distillation un appareillage susceptible de résister à la corrosion provoquée par les composés à séparer.

A cet effet, on choisit des matériaux avantageusement des aciers vitrifiés

On récupère un culot de distillation comprenant le sel alcalin de l'acide triflinique et les impuretés salines.

On peut effectuer la récupération du sel alcalin de l'acide triflinique présent dans la phase aqueuse obtenue suite à l'extraction liquide liquide ou dans le culot de distillation, selon différentes variantes d'exécution.

Selon un premier mode de réalisation de l'invention, on récupère le sel alcalin de l'acide triflinique présent dans la phase aqueuse obtenue suite à l'extraction liquide liquide ou dans le culot de distillation en les soumettant aux opérations suivantes :
- deuxième acidification de la phase récupérée de telle sorte qu'on libère le sel de l'acide triflinique sous sa forme acide,
- traitement de la phase aqueuse séparée comprenant essentiellement l'acide triflinique à l'aide d'un solvant organique susceptible d'extraire l'acide triflinique,
- séparation des phases organique et aqueuse,
- traitement de la phase organique à l'aide d'une solution aqueuse basique de telle sorte que l'on recueille le sel de l'acide triflinique en phase aqueuse,
- élimination de l'eau par distillation et remplacement par un solvant organique afin d'obtenir le sel de l'acide triflinique en solution organique,
- précipitation du sel de l'acide triflinique à l'aide d'un non solvant ou par cristallisation,
- séparation et récupération dudit sel.

On soumet la phase aqueuse recueillie à un deuxième traitement acide comme décrit ci-dessus à la seule différence que l'on met en oeuvre une quantité beaucoup plus forte d'acide. On libère ainsi le sel de l'acide triflinique sous sa forme acide.

La quantité d'acide fort mis en oeuvre est telle que le rapport en le nombre de moles d'acide exprimé en ions H⁺ et le nombre de moles de sel de l'acide triflinique varie entre 1 et 10 et de préférence entre 2 et 4.

Dans une étape suivante, on extrait l'acide triflinique à l'aide d'un solvant organique. On fait appel de préférence à un solvant organique basique comme décrit précédemment. On choisit de préférence les solvants de type éther et encore plus préférentiellement le méthyltertiobutyléther, le diisopropyléther, le vératrole.

En fin d'extraction, on obtient deux phases : une phase aqueuse comprenant les sels formés suite à l'acidification et une phase organique comprenant le solvant organique, l'acide triflinique et l'acide fort résiduaire.

Dans une étape suivante, on sépare les phases aqueuse et organique, selon les techniques classiques, décantation ou centrifugation.

On soumet la phase organique recueillie à un traitement basique afin de salifier l'acide triflinique obtenu.

A cet effet, on fait appel préférentiellement aux solutions basiques commerciales. Comme exemples, on peut mentionner les solutions aqueuses d'hydroxydes de métaux alcalins, de préférence de sodium ou de potassium ayant avantageusement une concentration de 10 % à 50 % en masse.

La quantité de base ajoutée est telle que l'on obtienne un pH compris entre 5 et 9, de préférence aux environs de 7.

En fin de traitement basique, on obtient deux phases : une phase aqueuse comprenant le sel de l'acide triflinique et une phase organique comprenant le solvant organique.

Dans une étape suivante, on sépare les phases aqueuse et organique, selon les techniques classiques, décantation ou centrifugation.

On soumet la phase aqueuse séparée à une opération de distillation ce qui permet d'éliminer l'eau en tête de distillation et de recueillir en pied de distillation essentiellement le sel de l'acide triflinique et moins de 2 à 10 % en masse d'eau.

A cet effet, on effectue la distillation à une température de bouilleur qui pour des raisons de sécurité est maintenue inférieure à 90°C.

La distillation est conduite préférentiellement à une température dans le bouilleur comprise entre 60°C et 90°C sous une pression comprise entre 1 mbar et 600 mbar.

Dans une étape suivante, on effectue l'élimination des traces d'eau par traitement du culot de distillation à l'aide d'un solvant organique.

Deux impératifs président au choix du solvant : celui doit être miscible à l'eau c'est-à-dire former un milieu homogène avec l'eau et il doit également solubiliser le sel de l'acide triflinique obtenu.

Comme exemples de solvants organiques répondant à ces exigences, on peut citer notamment les solvants de type alcool.

On préfère faire appel à des alcools ayant une faible condensation en carbone, de préférence, moins de 6 atomes de carbone et encore plus préférentiellement moins de 4 atomes de carbone.

Comme exemples plus spécifiques d'alcools convenant à la mise en oeuvre de l'invention, on peut citer entre autres, le méthanol, l'éthanol, le n-propanol, l'isopropanol, le butanol, l'isobutanol, le tert-butanol, l'alcool amylique, le cyclopentanol, le cyclohexanol, le dibenzylalcool.

L'isopropanol est l'alcool mis en oeuvre préférentiellement.

Comme autres solvants susceptibles d'être utilisés, on peut mentionner les solvants de type cétone comme par exemple la méthylisobutylcétone, la méthyléthylcétone ou les solvants de type ester tels que par exemple l'acétate d'éthyle ou de butyle.

On obtient une seule phase comprenant le solvant, le sel de l'acide triflinique et l'eau.

On peut éventuellement effectuer une filtration s'il y a présence de sels insolubles provenant de l'acide fort utilisé pour l'acidification extrait par le solvant organique.

A partir de la phase comprenant le solvant, le sel de l'acide triflinique et l'eau, on récupère le sel de l'acide triflinique selon deux modes de réalisation préférés : soit par précipitation par traitement de ladite phase à l'aide d'un non solvant, soit par cristallisation.

Selon le premier mode d'exécution, on précipite le sel de l'acide triflinique par traitement de la phase obtenue à l'aide d'un non-solvant.

Comme exemples de non-solvants, on peut citer notamment les hydrocarbures aliphatiques, cycloaliphatiques ou aromatiques et plus particulièrement, l'hexane, le cyclohexane, le méthylcyclohexane, les coupes pétrolières type éther de pétrole ; les hydrocarbures aromatiques comme notamment le toluène, les xylènes, le cumène, le mésitylène, les coupes pétrolières constituées de mélange d'alkylbenzènes notamment les coupes de type Solvesso.

En ce qui concerne les hydrocarbures halogénés aliphatiques ou aromatiques, on peut mentionner plus particulièrement, le dichlorométhane, le 1,2-dichloroéthane, le monochlorobenzène ou le dichlorobenzène.

On sépare le sel de l'acide triflinique, de préférence par filtration et l'on récupère une phase organique comprenant le solvant de type alcool et le non solvant.

On effectue éventuellement un ou plusieurs lavages effectués à l'aide du non-solvant tel que défini précédemment.

On effectue éventuellement un séchage à une température avantageusement comprise entre 20°C et 80°C, de préférence comprise entre 30°C et 50°C.

On conduit de préférence le séchage sous pression réduite choisie préférentiellement entre 0,1 et 200 mbar.

On conduit le séchage sous atmosphère contrôlée de gaz inertes tels que l'azote ou les gaz rares, par exemple l'argon.

On obtient le sel de l'acide triflinique purifié.

Selon l'autre mode de réalisation de l'invention, on récupère le sel de l'acide triflinique à partir de la phase comprenant le solvant, le sel de l'acide triflinique et l'eau, par cristallisation.

A cet effet, on le cristallise en effectuant un refroidissement de ladite phase par exemple entre 0°C et 40°C.

Le sel de l'acide triflinique cristallisé est séparé de préférence par filtration et séché comme précédemment décrit.

Selon un deuxième mode d'exécution de l'invention, on récupère le sel alcalin de l'acide triflinique présent dans la phase aqueuse en la soumettant aux opérations suivantes :
- concentration de la phase aqueuse séparée,
- traitement de la phase aqueuse séparée comprenant essentiellement le sel de l'acide triflinique, les sels formés suite à l'acidification et les formes acides correspondant aux impuretés salines à l'aide d'un solvant organique susceptible d'extraire le sel de l'acide triflinique,
- séparation des sels en suspension,
- séparation des phases organique et aqueuse,
- récupération du sel de l'acide triflinique à partir de la phase organique séparée.

On soumet la phase aqueuse comprenant le sel de l'acide triflinique qui est recueillie suite à la séparation des phases organique et aqueuse, à une opération de concentration.

Selon une caractéristique du procédé de l'invention, on peut effectuer une concentration du milieu réactionnel de façon à augmenter la concentration du sel de l'acide triflinique de telle sorte qu'il y ait présence entre 2 et 30 %, de préférence entre 5 et 20 % d'eau dans la solution concentrée obtenue.

Un autre mode de réalisation pour concentrer le milieu réactionnel consiste à effectuer la distillation de la quantité d'une partie de l'eau pour atteindre dans le milieu réactionnel la concentration souhaitée en acide triflinique sous forme salifiée.

On peut effectuer la distillation sous pression atmosphérique à une température à 100°C.

On peut également effectuer la distillation sous une pression légèrement inférieure à la pression atmosphérique, par exemple comprise entre 5 mbar et 600 mbar et à une température inférieure à 100°C. En général, la pression est choisie pour avoir une température de distillation située entre 40°C et 90°C.

Un autre mode consiste à effectuer un entraînement par injection d'un fluide, par exemple vapeur ou gaz inerte, notamment azote.

D'un point de vue pratique, les opérations de concentration de même que les opérations de distillation décrites ci-avant peuvent être conduites dans un évaporateur. On peut utiliser ceux qui sont disponibles dans le commerce et l'on peut citer entre autres, les évaporateurs à film raclé ou tombant de type LUWA^{®}_{.}

L'invention n'exclut pas la mise en oeuvre d'autres techniques de concentration telles que l'ultrafiltration ou l'osmose inverse.

Dans une étape suivante, on extrait le sel de l'acide triflinique à l'aide d'un solvant organique. On fait appel de préférence à un solvant organique aprotique polaire et insoluble ou partiellement soluble dans l'eau (par exemple moins de 5 % en masse).

On choisit de préférence les solvants de type alcool comme le méthanol, l'éthanol, le propanol, l'isopropanol, le butanol, l'isobutanol, le tert-butanol, l'alcool amylique, le cyclopentanol, le cyclohexanol, le dibenzylalcool ou de type cétone comme la méthylisobutylcétone ou la méthyléthylcétone ou de type ester comme l'acétate de méthyle, d'éthyle, de propyle, d'isopropyle, de butyle, d'isobutyle, de tert-butyle, d'isoamyle. Cette liste de solvants n'est en aucun cas limitative.

En fin d'extraction, on obtient une phase organique comprenant essentiellement le sel de l'acide triflinique et une phase aqueuse comprenant les sels formés suite à l'acidification et les formes acides correspondant aux impuretés salines.

On sépare les phases aqueuse et organique.

On concentre la phase organique par évaporation du solvant organique de telle sorte que le sel de l'acide triflinique précipite.

A cet effet, on porte la solution organique obtenue à une température inférieure à 90°C, de préférence comprise entre 30°C et 60°C.

On laisse refroidir à la température ambiante : le sel de l'acide triflinique précipite.

On sépare le sel de l'acide triflinique, selon les techniques classiques de séparation solide/liquide de préférence par filtration.

On peut effectuer un ou plusieurs lavages effectués à l'aide du solvant précédemment utilisé de type alcool, cétone ou ester ou à l'aide d'un non-solvant qui peut être choisi parmi ceux listés ci-dessus.

Dans une étape suivante, on peut soumettre le sel d'acide triflinique obtenu à une opération de séchage qui peut être conduite comme indiqué ci-dessus pour la première variante d'exécution de l'invention.

On obtient le sel de l'acide triflinique sous une forme purifiée.

Selon un troisième mode de réalisation de l'invention, on récupère le sel alcalin de l'acide triflinique présent dans la phase aqueuse en la soumettant aux opérations suivantes :
- élimination de l'eau présente dans le phase aqueuse comprenant le sel alcalin de l'acide triflinique,
- ajout d'un solvant organique pour solubiliser le sel de l'acide triflinique,
- récupération du sel de l'acide triflinique à partir de la phase organique séparée.

Conformément à ce mode de réalisation, on peut éliminer l'eau par simple distillation de l'eau conduite à une température de 100°C ou à une température inférieure lorsque la pression est choisie inférieure à la pression atmosphérique mais il est préférable d'éliminer l'eau par distillation azéotropique grâce à la mise en oeuvre d'un solvant susceptible de former un azéotrope avec l'eau et également susceptible de solubiliser le sel de l'acide triflinique.

Comme solvants convenant tout à fait bien, on peut citer notamment les solvants de type alcool et plus particulièrement le méthanol, l'éthanol, les propanols, les butanols, l'alcool amylique, le cyclopentanol, le cyclohexanol, le dibenzylalcool. Parmi ces alcools, les butanols sont préférés.

La distillation est généralement conduite avantageusement à une température dans le bouilleur comprise entre 40°C et 70°C, et sous une pression réduite inférieure à 1 bar est supérieure à 10 mbar, de préférence comprise entre 15 mbar et 400 mbar.

En fin de distillation, on récupère en pied de distillation, le sel de l'acide triflinique solubilisé dans le solvant organique.

On récupère ledit sel d'une manière telle que décrite précédemment, par exemple par précipitation par addition d'un non solvant, ou plus simplement par cristallisation par refroidissement.

On sépare le sel de l'acide triflinique, selon les techniques classiques de séparation solide/liquide de préférence par filtration.

On peut le soumettre à une opération de séchage comme précédemment décrite.

On obtient le sel de l'acide triflinique sous une forme purifiée.

Le procédé de l'invention est très intéressant car il conduit à un sel de l'acide triflinique d'une grande pureté, supérieure ou égale à 90 %, de préférence supérieure ou égale à 95 % et comprise préférentiellement entre 95 et 98 %.

On donne ci-après des exemples de réalisation de l'invention donnés à titre indicatif et sans caractère limitatif.

### Exemple 1

Une solution aqueuse (1,7 kg) contenant 16 % en masse de trifluorométhanesulfinate de potassium et à 15 % en masse de trifluoroacétate de potassium est chargée dans un réacteur à double enveloppe en verre équipé d'une agitation centrale et maintenu sous atmosphère d'azote.

De l'acide sulfurique (163 g) concentré à 96 % est ajouté en maintenant la température de la masse réactionnelle inférieure à 10°C.

Un précipité apparaît, qui est aisément éliminé par filtration.

On obtient ainsi 1750 g de solution aqueuse.

Quatre extractions liquide / liquide de la phase aqueuse sont réalisées avec 4 430 g de méthyltertiobutyléther.

Les phases organiques obtenues sont éliminées et la phase aqueuse restante (1 440 g) est acidifiée avec 375 g d'acide sulfurique concentré à 96 %.

La phase aqueuse est extraite trois fois avec 1720 g de méthyltertiobutyléther.

Les phases organiques sont réunies (m = 1560 g) et la phase aqueuse résiduelle (m = 1538 g) est éliminée.

Les solutions organiques précédentes sont neutralisées à 0°C à l'aide de 138 g d'une solution de potasse concentrée à 50 % en masse.

En fin de neutralisation, la phase aqueuse est proche de la neutralité.

On obtient ainsi 456 g de solution aqueuse.

Cette solution est concentrée sous une pression réduite de 20 mbar et une température de 50°C environ de façon à éliminer 241 g d'eau.

De l'isopropanol est ajouté (320 g) et la distillation est maintenue dans des conditions identiques jusqu'à obtenir 521 g de solution organique.

La solution organique est coulée sur 1100 g de toluène anhydre.

Le solide précipité est isolé par filtration et séché sous une pression de 10 mbar à 50°C.

On obtient 184 g de trifluorométhanesulfinate de potassium à 97 % de pureté.

### Exemple 2

Une solution aqueuse (1,7 kg) contenant 16 % en masse de trifluorométhanesulfinate de potassium et 15 % en masse de trifluoroacétate de potassium est chargée dans un réacteur.

De l'acide sulfurique (163 g) concentré à 96 % est ajouté en maintenant la température de la masse réactionnelle inférieure à 10°C.

Un précipité apparaît, qui est aisément éliminé par filtration.

On obtient ainsi 1750 g de solution aqueuse.

Quatre extractions liquide / liquide de la phase aqueuse sont réalisées avec 4 430 g de méthyltertiobutyléther.

Les phases organiques obtenues sont éliminées et la phase aqueuse restante (1440 g) est acidifiée avec 375 g d'acide sulfurique concentré à 96 %.

Cette phase aqueuse est concentrée sous une pression réduite de 3 mbar et une température de 45°C de façon à distiller 186 g d'eau.

La solution aqueuse concentrée obtenue (m = 353 g) est diluée dans 630 g d'alcool isopropylique.

Il se forme un précipité qui est éliminé par filtration.

On obtient ainsi 778 g d'une solution organique homogène.

Cette solution est concentrée sous une pression réduite de 20 mbar et une température de 40°C de façon à distiller 230 g d'alcool isopropylique.

Après concentration, la solution organique est coulée sur 910 g de toluène, faisant précipiter le trifluorométhanesulfinate de potassium.

Le solide est isolé par filtration, et séché à 50°C sous une pression de 10 mbar.

On obtient 175 g de trifluorométhanesulfinate de potassium sec à 96 % de pureté.

### Exemple 3

Une solution aqueuse (1,7 kg) contenant 16 % en masse de trifluorométhanesulfinate de potassium et 15 % en masse de trifluoroacétate de potassium est chargée dans un réacteur.

De l'acide sulfurique (163 g) concentré à 96 % est ajouté en maintenant la température de la masse réactionnelle inférieure à 10°C.

Un précipité apparaît, qui est aisément éliminé par filtration.

On obtient ainsi 1750 g de solution aqueuse.

Quatre extractions liquide / liquide de la phase aqueuse sont réalisées avec 4 430 g de méthyltertiobutyléther.

Les phases organiques obtenues sont éliminées et la phase aqueuse restante (1 440 g) est acidifiée avec 375 g d'acide sulfurique concentré à 96%.

Cette phase aqueuse est concentrée sous une pression réduite de 3 mbar et une température de 45°C, de façon à distiller 186 g d'eau.

La solution aqueuse concentrée obtenue (m = 353 g) est diluée dans 630 g d'alcool isopropylique.

Il se forme un précipité qui est éliminé par filtration.

On obtient ainsi 785 g d'une solution organique homogène.

Cette solution est concentrée sous une pression de 20 mbar et une température de 40°C de façon à obtenir 291 g de solution organique concentrée.

La température est abaissée à l'ambiante et un solide cristallise.

Celui-ci est isolé par filtration, et séché à 45°C sous une pression de 2 mbar.

On obtient 133 g de trifluorométhanesulfinate de potassium sec à 96 % de pureté.

### Exemple 4

Une solution aqueuse (100 g) contenant 16 % en masse de trifluorométhanesulfinate de potassium et 15 % en masse de trifluoroacétate de potassium est chargée dans un réacteur.

De l'acide sulfurique (9,5 g, 0,09 mol) est ajouté en maintenant la température à 10°C.

Un solide apparait et celui-ci est éliminé par filtration.

On obtient 14,6 g de solide et 90,4 g de filtrat.

Ce filtrat est ensuite distillé sous pression réduite.

La température est portée à 70°C sous une pression réduite de 20 mbar.

De l'eau est ajoutée pour maintenir le niveau de liquide constant dans le bouilleur.

On obtient ainsi une fraction de 92 g contenant 16 % en masse d'acide trifluoroacétique et 84 % en masse d'eau.

De l'isobutanol (800 g) est ajouté au milieu et l'eau est éliminée par distillation azéotropique sous une pression de 120 mbar en portant la température du milieu à 60°C.

La fraction ayant un point d'ébullition de 42°C est isolée.

Au fur et à mesure de l'élimination de l'eau, un solide blanc apparaît.

Celui-ci est éliminé du milieu par filtration.

On obtient 12,9 g de solide.

La distillation azéotropique est stoppée lorsque 774 g de distillat est obtenu.

La température est abaissée à 0°C et un solide cristallise.

Celui-ci est isolé par filtration sous atmosphère inerte, puis essoré sous un flux d'azote.

On obtient 39,6 g de solide blanc.

Celui-ci est séché sous une pression de 0,1 mbar à température ambiante pendant 72 heures.

On obtient 32,8 g de trifluorométhanesulfinate de potassium ayant une pureté de 96 % en masse.

## Revendications

1. - Procédé de préparation d'un sel de l'acide triflinique de haute pureté, à partir d'un mélange aqueux le comprenant associé à un sel d'acide trifluoroacétique et des impuretés salines résultant de son procédé de préparation, **caractérisé par le fait que** ledit mélange est soumis aux opérations suivantes :
- première acidification contrôlée de telle sorte que l'on libère essentiellement le sel de l'acide trifluoroacétique sous sa forme acide, la majorité de l'acide triflinique restant sous une forme salifiée,
- séparation éventuelle des sels provenant de l'acide ayant servi à l'acidification et récupération d'une phase aqueuse,
- séparation de l'acide trifluoroacétique à partir de la phase aqueuse séparée comprenant le sel alcalin de l'acide triflinique, l'acide trifluoroacétique, l'acide triflinique, l'acide fort en excès, conduisant alors à une phase aqueuse épuisée en acide trifluoroacétique mais comprenant le sel alcalin de l'acide triflinique,
- récupération du sel alcalin de l'acide triflinique à partir de la phase aqueuse.

2. - Procédé selon la revendication 1 **caractérisé par le fait que** le mélange aqueux est une solution aqueuse de 10 à 40 % en masse de matières sèches comprenant :
- de 5 à 35 % en masse d'un sel d'acide triflinique, de préférence un sel alcalin,
- de 5 à 35 % en masse d'un sel d'acide trifluoroacétique, de préférence un sel alcalin,
- de 0,5 à 2 % en masse d'impuretés salines.

3. - Procédé selon l'une des revendications 1 et 2 **caractérisé par le fait que** l'on effectue l'acidification à l'aide d'acide sulfurique, chlorhydrique ou phosphorique, un oléum ou de l'acide chlorhydrique gazeux.

4. - Procédé selon la revendication 3 **caractérisé par le fait que** la quantité d'acide fort mis en oeuvre est telle que le rapport entre le nombre de moles d'acide exprimé en ions H⁺ et le nombre de moles du sel d'acide trifluoroacétique varie entre 1 et 3, de préférence entre 1 et 2.

5. - Procédé selon l'une des revendications 3 et 4 **caractérisé par le fait que** l'on recueille en fin de traitement acide, une solution ou une suspension aqueuse comprenant le sel de l'acide triflinique, l'acide trifluoroacétique, l'acide triflinique, l'acide fort en excès, les sels formés suite à l'acidification et les formes acides correspondant aux impuretés salines.

6. - Procédé selon la revendication 1 **caractérisé par le fait que** l'on ajoute dans la solution ou la suspension aqueuse obtenue avant l'extraction des acides organiques, un composé susceptible de piéger l'acide fluorhydrique.

7. - Procédé selon la revendication 1 **caractérisé par le fait que** la séparation de l'acide trifluoroacétique concomitamment avec l'acide triflinique est faite par traitement de la phase aqueuse séparée comprenant le sel alcalin de l'acide triflinique, l'acide trifluoroacétique, l'acide triflinique, l'acide fort en excès à l'aide d'un solvant organique susceptible d'extraire les acides organiques formés (acide trifluoroacétique, acide triflinique) suivi par la séparation des phases organique et aqueuse.

8. - Procédé selon la revendication 7 **caractérisé par le fait que** l'on procède au traitement de la phase aqueuse obtenue à l'aide d'un solvant organique de type amine ou éther.

9. - Procédé selon l'une des revendications 7 et 8 **caractérisé par le fait que** l'on obtient en fin d'extraction, deux phases : une phase aqueuse comprenant le sel de l'acide triflinique et éventuellement des sels formés suite à l'acidification et une phase organique comprenant le solvant organique, les acides organiques formés et éventuellement l'excès de l'acide utilisé à l'acidification.

10. - Procédé selon la revendication 1 **caractérisé par le fait que** la séparation de l'acide trifluoroacétique est faite par distillation conduite sur la phase aqueuse séparée comprenant le sel alcalin de l'acide triflinique, l'acide trifluoroacétique, l'acide triflinique, l'acide fort en excès permettant d'éliminer en tête de distillation, l'acide trifluoroacétique et éventuellement de l'eau et de récupérer en pied de distillation le sel de l'acide triflinique accompagné de l'acide fort en excès, des sels des acides forts notamment ceux provenant de l'acidification et de l'eau.

11. - Procédé selon la revendication 10 **caractérisé par le fait que** la distillation est conduite à une température dans le bouilleur comprise entre 60°C et 90°C sous une pression allant de 700 mbar à 10 mbar.

12. - Procédé selon l'une des revendications 9 et 10 **caractérisé par le fait que** l'on récupère le sel alcalin de l'acide triflinique présent dans la phase aqueuse ou dans le culot de distillation en les soumettant aux opérations suivantes :
- deuxième acidification de la phase récupérée de telle sorte qu'on libère le sel de l'acide triflinique sous sa forme acide,
- traitement de la phase aqueuse séparée comprenant essentiellement l'acide triflinique à l'aide d'un solvant organique susceptible d'extraire l'acide triflinique,
- séparation des phases organique et aqueuse,
- traitement de la phase organique à l'aide d'une solution aqueuse basique de telle sorte que l'on recueille le sel de l'acide triflinique en phase aqueuse,
- élimination de l'eau par distillation et remplacement par un solvant organique afin d'obtenir le sel de l'acide triflinique en solution organique,
- précipitation du sel de l'acide triflinique à l'aide d'un non solvant ou par cristallisation,
- séparation et récupération dudit sel.

13. - Procédé selon la revendication 12 **caractérisé par le fait que** l'on soumet la phase aqueuse recueillie à un deuxième traitement acide à l'aide d'un acide fort en une quantité telle que le rapport entre le nombre de moles d'acide exprimé en ions H⁺ et le nombre de moles de sel de l'acide triflinique varie entre 1 et 10 et de préférence entre 2 et 4.

14. - Procédé selon la revendication 12 **caractérisé par le fait que** l'on extrait l'acide triflinique à l'aide d'un solvant organique, de préférence le méthyltertiobutyléther, le diisopropyléther, le vératrole.

15. - Procédé selon la revendication 12 **caractérisé par le fait que** l'on obtient en fin d'extraction, deux phases : une phase aqueuse comprenant les sels formés suite à l'acidification et une phase organique comprenant le solvant organique, l'acide triflinique et l'acide fort résiduaire.

16. - Procédé selon la revendication 12 **caractérisé par le fait que** l'on soumet la phase organique recueillie à un traitement basique afin de salifier l'acide triflinique obtenu : la quantité de base ajoutée étant telle que l'on obtienne un pH compris entre 5 et 9.

17. - Procédé selon la revendication 16 **caractérisé par le fait que** l'on obtient en fin de traitement basique deux phases : une phase aqueuse comprenant le sel de l'acide triflinique et le sel formé suite l'acidification et une phase organique comprenant le solvant organique.

18. - Procédé selon la revendication 12 **caractérisé par le fait que** l'on soumet la phase aqueuse séparée à une opération de distillation à une température dans le bouilleur comprise entre 60°C et 90°C sous une pression comprise entre 1 mbar et 600 mbar.

19. - Procédé selon la revendication 12 **caractérisé par le fait que** l'on traite le culot de distillation à l'aide d'un solvant organique de type alcool, cétone ou ester, de préférence l'isopropanol.

20. - Procédé selon la revendication 12 **caractérisé par le fait que** l'on précipite le sel de l'acide triflinique par traitement de la phase obtenue à l'aide d'un non-solvant, de préférence un hydrocarbure aliphatique, cycloaliphatique ou aromatique ; un hydrocarbure halogéné aliphatique ou aromatique.

21. - Procédé selon la revendication 12 **caractérisé par le fait que** l'on sépare le sel de l'acide triflinique, de préférence par filtration et l'on récupère une phase organique comprenant le solvant de type alcool et le non solvant.

22. - Procédé selon la revendication 1 **caractérisé par le fait que** l'on récupère le sel alcalin de l'acide triflinique présent dans la phase aqueuse en la soumettant aux opérations suivantes :
- concentration de la phase aqueuse séparée,
- traitement de la phase aqueuse séparée comprenant essentiellement le sel de l'acide triflinique, les sels formés suite à l'acidification et les formes acides correspondant aux impuretés salines, à l'aide d'un solvant organique susceptible d'extraire le sel de l'acide triflinique,
- séparation des sels en suspension,
- séparation des phases organique et aqueuse,
- récupération du sel de l'acide triflinique à partir de la phase organique séparée.

23. - Procédé selon la revendication 22 **caractérisé par le fait que** l'on effectue une concentration du milieu réactionnel de façon à augmenter la concentration du sel de l'acide triflinique de telle sorte qu'il y ait présence entre 2 et 30 %, de préférence entre 5 et 20 % d'eau dans la solution concentrée obtenue.

24. - Procédé selon la revendication 23 **caractérisé par le fait que** l'on élimine de l'eau par distillation sous pression atmosphérique ou inférieure à la pression atmosphérique ou par injection d'un fluide vapeur ou gaz inerte.

25. - Procédé selon la revendication 22 **caractérisé par le fait que** l'on extrait le sel de l'acide triflinique à l'aide d'un solvant organique de type alcool, cétone ou ester.

26. - Procédé selon la revendication 25 **caractérisé par le fait que** l'on obtient en fin d'extraction, une phase organique comprenant essentiellement le sel de l'acide triflinique et une phase aqueuse comprenant les sels formés suite à l'acidification et les formes acides correspondant aux impuretés salines.

27. - Procédé selon la revendication 26 **caractérisé par le fait que** l'on sépare les phases aqueuse et organique et l'on concentre la phase organique par évaporation du solvant organique de telle sorte que le sel de l'acide triflinique précipite.

28. - Procédé selon la revendication 1 **caractérisé par le fait que** l'on récupère le sel alcalin de l'acide triflinique présent dans la phase aqueuse en la soumettant aux opérations suivantes :
- élimination de l'eau présente dans le phase aqueuse comprenant le sel alcalin de l'acide triflinique,
- ajout d'un solvant organique pour solubiliser le sel de l'acide triflinique,
- récupération du sel de l'acide triflinique à partir de la phase organique séparée.

29. - Procédé selon la revendication 28 **caractérisé par le fait que** l'eau est éliminée par distillation azéotropique effectuée en présence d'un solvant de préférence de type alcool.

30. - Procédé selon la revendication 28 **caractérisé par le fait que** le sel de l'acide triflinique est cristallisé par refroidissement puis séparé selon les techniques classiques de séparation solide/liquide de préférence par filtration.

31. - Procédé selon l'une des revendications 1 à 30 **caractérisé par le fait que** l'on effectue un séchage à une température comprise entre 20°C et 80°C, de préférence comprise entre 30°C et 50°C sous une pression de préférence réduite choisie entre 0,1 et 200 mbar et de préférence sous atmosphère contrôlée de gaz inertes plus préférentiellement l'azote ou l'argon.

32. - Procédé selon l'une des revendications 1 à 31 **caractérisé par le fait que** le sel de l'acide triflinique obtenu a une pureté supérieure ou égale à 90 %, de préférence supérieure ou égale à 95 % et comprise préférentiellement entre 95 et 98 %.

## Claims

1. Process for the preparation of a triflinic acid salt of high purity starting from an aqueous mixture comprising it in combination with a trifluoroacetic acid salt and saline impurities resulting from its process of preparation, **characterized in that** said mixture is subjected to the following operations:
- first acidification, controlled in such a way that essentially the salt of the trifluoroacetic acid is released in its acid form, the majority of the triflinic acid remaining in a salified form,
- optional separation of the salts originating from the acid which has been used for the acidification and recovery of an aqueous phase,
- separation of the trifluoroacetic acid from the separated aqueous phase comprising the alkali metal salt of the triflinic acid, the trifluoroacetic acid, the triflinic acid and the excess strong acid, then resulting in an aqueous phase impoverished in trifluoroacetic acid but comprising the alkali metal salt of the triflinic acid,
- recovery of the alkali metal salt of the triflinic acid from the aqueous phase.

2. Process according to Claim 1, **characterized in that** the aqueous mixture is an aqueous solution with a solids content of 10 to 40% by weight comprising:
- from 5 to 35% by weight of a triflinic acid salt, preferably an alkali metal salt,
- from 5 to 35% by weight of a trifluoroacetic acid salt, preferably an alkali metal salt,
- from 0.5 to 2% by weight of saline impurities.

3. Process according to either of Claims 1 and 2, **characterized in that** the acidification is carried out using sulfuric, hydrochloric or phosphoric acid, an oleum or gaseous hydrochloric acid.

4. Process according to Claim 3, **characterized in that** the amount of strong acid employed is such that the ratio of the number of moles of acid, expressed as H⁺ ions, to the number of moles of the trifluoroacetic acid salt varies between 1 and 3, preferably between 1 and 2.

5. Process according to either of Claims 3 and 4, **characterized in that**, at the end of the acid treatment, an aqueous solution or suspension is collected which comprises the salt of the triflinic acid, the trifluoroacetic acid, the triflinic acid, the excess strong acid, the salts formed subsequent to the acidification and the acid forms corresponding to the saline impurities.

6. Process according to Claim 1, **characterized in that** a compound capable of trapping hydrofluoric acid is added to the aqueous solution or suspension obtained before the extraction of the organic acids.

7. Process according to Claim 1, **characterized in that** the separation of the trifluoroacetic acid concomitantly with the triflinic acid is carried out by treatment of the separated aqueous phase, comprising the alkali metal salt of the triflinic acid, the trifluoroacetic acid, the triflinic acid and the excess strong acid, using an organic solvent capable of extracting the organic acids formed (trifluoroacetic acid, triflinic acid), followed by the separation of the organic and aqueous phases.

8. Process according to Claim 7, **characterized in that** the aqueous phase obtained is treated using an organic solvent of amine or ether type.

9. Process according to either of Claims 7 and 8, **characterized in that** two phases are obtained at the end of the extraction: an aqueous phase, comprising the salt of the triflinic acid and optionally salts formed subsequent to the acidification, and an organic phase, comprising the organic solvent, the organic acids formed and optionally the excess of the acid used in the acidification.

10. Process according to Claim 1, **characterized in that** the trifluoroacetic acid is separated by distillation carried out on the separated aqueous phase, comprising the alkali metal salt of the triflinic acid, the trifluoroacetic acid, the triflinic acid and the excess strong acid, making it possible to remove, at the distillation top, the trifluoroacetic acid and optionally water and to recover, at the distillation bottom, the salt of the triflinic acid, accompanied by the excess strong acid, the salts of the strong acids, in particular those originating from the acidification, and water.

11. Process according to Claim 10, **characterized in that** the distillation is carried out at a temperature in the reboiler of between 60°C and 90°C under a pressure ranging from 700 mbar to 10 mbar.

12. Process according to either of Claims 9 and 10, **characterized in that** the alkali metal salt of the triflinic acid present in the aqueous phase or in the distillation concentrate is recovered by subjecting them to the following operations:
- second acidification of the recovered phase in such a way that the salt of the triflinic acid is released in its acid form,
- treatment of the separated aqueous phase comprising essentially the triflinic acid using an organic solvent capable of extracting the triflinic acid,
- separation of the organic and aqueous phases,
- treatment of the organic phase using a basic aqueous solution in such a way that the salt of the triflinic acid is collected in the aqueous phase,
- removal of the water by distillation and replacement with an organic solvent in order to obtain the salt of the triflinic acid in an organic solution,
- precipitation of the salt of the triflinic acid using a nonsolvent or by crystallization,
- separation and recovery of said salt.

13. Process according to Claim 12, **characterized in that** the aqueous phase collected is subjected to a second acid treatment using a strong acid in an amount such that the ratio of the number of moles of acid, expressed as H⁺ ions, to the number of moles of salt of the triflinic acid varies between 1 and 10 and preferably between 2 and 4.

14. Process according to Claim 12, **characterized in that** the triflinic acid is extracted using an organic solvent, preferably methyl tert-butyl ether, diisopropyl ether or veratrole.

15. Process according to Claim 12, **characterized in that** two phases are obtained at the end of the extraction: an aqueous phase, comprising the salts formed subsequent to the acidification, and an organic phase, comprising the organic solvent, the triflinic acid and the residual strong acid.

16. Process according to Claim 12, **characterized in that** the organic phase collected is subjected to a basic treatment in order to salify the triflinic acid obtained: the amount of base added being such that a pH of between 5 and 9 is obtained.

17. Process according to Claim 16, **characterized in that** two phases are obtained at the end of the basic treatment: an aqueous phase, comprising the salt of the triflinic acid and the salt formed subsequent to the acidification, and an organic phase, comprising the organic solvent.

18. Process according to Claim 12, **characterized in that** the separated aqueous phase is subjected to a distillation operation at a temperature in the reboiler of between 60°C and 90°C under a pressure of between 1 mbar and 600 mbar.

19. Process according to Claim 12, **characterized in that** the distillation concentrate is treated using an organic solvent of alcohol, ketone or ester type, preferably isopropanol.

20. Process according to Claim 12, **characterized in that** the salt of the triflinic acid is precipitated by treatment of the phase obtained using a nonsolvent, preferably an aliphatic, cycloaliphatic or aromatic hydrocarbon; or a halogenated aliphatic or aromatic hydrocarbon.

21. Process according to Claim 12, **characterized in that** the salt of the triflinic acid is separated, preferably by filtration, and an organic phase is recovered which comprises the solvent of alcohol type and the nonsolvent.

22. Process according to Claim 1, **characterized in that** the alkali metal salt of the triflinic acid present in the aqueous phase is recovered by subjecting the aqueous phase to the following operations:
- concentration of the separated aqueous phase,
- treatment of the separated aqueous phase, comprising essentially the salt of the triflinic acid, the salts formed subsequent to the acidification and the acid forms corresponding to the saline impurities, using an organic solvent capable of extracting the salt of the triflinic acid,
- separation of the suspended salts,
- separation of the organic and aqueous phases,
- recovery of the salt of the triflinic acid from the separated organic phase.

23. Process according to Claim 22, **characterized in that** the reaction medium is concentrated so as to increase the concentration of the salt of the triflinic acid in such a way that between 2 and 30%, preferably between 5 and 20%, of water is present in the concentrated solution obtained.

24. Process according to Claim 23, **characterized in that** water is removed by distillation at atmospheric pressure or under a pressure which is less than atmospheric pressure or by injection of a steam or inert gas fluid.

25. Process according to Claim 22, **characterized in that** the salt of the triflinic acid is extracted using an organic solvent of alcohol, ketone or ester type.

26. Process according to Claim 25, **characterized in that**, at the end of the extraction, an organic phase, comprising essentially the salt of the triflinic acid, and an aqueous phase, comprising the salts formed subsequent to the acidification and the acid forms corresponding to the saline impurities, are obtained.

27. Process according to Claim 26, **characterized in that** the aqueous and organic phases are separated and the organic phase is concentrated by evaporation of the organic solvent in such a way that the salt of the triflinic acid precipitates.

28. Process according to Claim 1, **characterized in that** the alkali metal salt of the triflinic acid present in the aqueous phase is recovered by subjecting the aqueous phase to the following operations:
- removal of the water present in the aqueous phase comprising the alkali metal salt of the triflinic acid,
- addition of an organic solvent in order to dissolve the salt of the triflinic acid,
- recovery of the salt of the triflinic acid from the separated organic phase.

29. Process according to Claim 28, **characterized in that** the water is removed by azeotropic distillation carried out in the presence of a solvent, preferably of alcohol type.

30. Process according to Claim 28, **characterized in that** the salt of the triflinic acid is crystallized by cooling and then separated according to conventional solid/liquid separation techniques, preferably by filtration

31. Process according to one of Claims 1 to 30, **characterized in that** drying is carried out at a temperature of between 20°C and 80°C, preferably of between 30°C and 50°C, under a preferably reduced pressure chosen between 0.1 and 200 mbar and preferably under a controlled atmosphere of inert gases, more preferably nitrogen or argon.

32. Process according to one of Claims 1 to 31, **characterized in that** the salt of the triflinic acid obtained has a purity of greater than or equal to 90%, preferably of greater than or equal to 95% and preferentially of between 95 and 98%.

## Patentansprüche

1. Verfahren zur Herstellung eines hochreinen Trifluormethansulfinsäuresalzes ausgehend von einer wässrigen Mischung, die es zusammen mit einem Trifluoressigsäuresalz und aus dem Herstellungsverfahren stammenden Salzverunreinigungen umfasst, **dadurch gekennzeichnet, dass** man die Mischung den folgenden Arbeitsgängen unterwirft:
- erstes kontrolliertes Ansäuern derart, dass das Trifluoressigsäuresalz im Wesentlichen in seiner Säureform freigesetzt wird, wobei der größte Teil der Trifluormethansulfinsäure in versalzter Form verbleibt,
- gegebenenfalls Abtrennen der von der zum Ansäuern verwendeten Säure stammenden Salze und Gewinnen einer wässrigen Phase,
- Abtrennen der Trifluoressigsäure aus der abgetrennten wässrigen Phase, die das Alkalimetallsalz der Trifluormethansulfinsäure, die Trifluoressigsäure, die Trifluormethansulfinsäure und die überschüssige starke Säure umfasst, was dann eine wässrige Phase ergibt, die an Trifluoressigsäure abgereichert ist, aber das Alkalimetallsalz der Trifluormethansulfinsäure umfasst,
- Gewinnen des Alkalimetallsalzes der Trifluormethansulfinsäure aus der wässrigen Phase.

2. Verfahren nach Anspruch 1, **dadurch gekennzeichnet, dass** es sich bei der wässrigen Mischung um eine wässrige Lösung mit einem Feststoffgehalt von 10 bis 40 Gew.-% handelt, die
- 5 bis 35 Gew.-% eines Trifluormethansulfinsäuresalzes, vorzugsweise eines Alkalimetallsalzes,
- 5 bis 35 Gew.-% eines Trifluoressigsäuresalzes, vorzugsweise eines Alkalimetallsalzes,
- 0,5 bis 2 Gew.-% Salzverunreinigungen umfasst.

3. Verfahren nach einem der Ansprüche 1 und 2, **dadurch gekennzeichnet, dass** man das Ansäuern mit Hilfe von Schwefelsäure, Salzsäure oder Phosphorsäure, Oleum oder gasförmiger Salzsäure durchführt.

4. Verfahren nach Anspruch 3, **dadurch gekennzeichnet, dass** die eingesetzte Menge an starker Säure derart beschaffen ist, dass das Verhältnis zwischen der Zahl der Mole Säure, ausgedrückt als H⁺-Ionen, und der Zahl der Mole des Trifluoressigsäuresalzes zwischen 1 und 3, vorzugsweise zwischen 1 und 2, variiert.

5. Verfahren nach einem der Ansprüche 3 und 4, **dadurch gekennzeichnet, dass** man am Ende der Säurebehandlung eine wässrige Lösung oder Suspension, die das Trifluormethansulfinsäuresalz, die Trifluoressigsäure, die Trifluormethansulfinsäure, die überschüssige starke Säure, die nach dem Ansäuern gebildeten Salze und die den Salzverunreinigungen entsprechenden Säureformen umfasst, gewinnt.

6. Verfahren nach Anspruch 1, **dadurch gekennzeichnet, dass** man die erhaltene wässrige Lösung oder Suspension vor der Extraktion der organischen Säuren mit einer zum Abfangen von Fluorwasserstoffsäure befähigten Verbindung versetzt.

7. Verfahren nach Anspruch 1, **dadurch gekennzeichnet, dass** man das Abtrennen der Trifluoressigsäure zusammen mit der Trifluormethansulfinsäure durchführt, indem man die abgetrennte wässrige Phase, die das Alkalimetallsalz der Trifluormethansulfinsäure, die Trifluoressigsäure, die Trifluormethansulfinsäure und die überschüssige starke Säure umfasst, mit einem organischen Lösungsmittel, das zum Extrahieren der gebildeten organischen Säuren (Trifluoressigsäure, Trifluormethansulfinsäure) befähigt ist, behandelt und danach die organische Phase und die wässrige Phase trennt.

8. Verfahren nach Anspruch 7, **dadurch gekennzeichnet, dass** man die erhaltene wässrige Phase mit einem organischen Lösungsmittel vom Amin- oder Ether-Typ behandelt.

9. Verfahren nach einem der Ansprüche 7 und 8, **dadurch gekennzeichnet, dass** man am Ende der Extraktion zwei Phasen erhält: eine wässrige Phase, die das Trifluormethansulfinsäuresalz und gegebenenfalls nach dem Ansäuern gebildete Salze umfasst, und eine organische Phase, die das organische Lösungsmittel, die gebildeten organischen Säuren und gegebenenfalls den Überschuss der beim Ansäuern verwendeten Säure umfasst.

10. Verfahren nach Anspruch 1, **dadurch gekennzeichnet, dass** das Abtrennen der Trifluoressigsäure durch Destillation der abgetrennten wässrigen Phase, die das Alkalimetallsalz der Trifluormethansulfinsäure, die Trifluoressigsäure, die Trifluormethansulfinsäure und die überschüssige starke Säure umfasst, erfolgt, wodurch am Kopf der Destillation die Trifluoressigsäure und gegebenenfalls Wasser
eliminiert und am Sumpf der Destillation das Trifluormethansulfinsäuresalz zusammen mit der überschüssigen starken Säure, den Salzen der starken Säuren, insbesondere den beim Ansäuern angefallenen Salzen, und Wasser gewonnen werden kann.

11. Verfahren nach Anspruch 10, **dadurch gekennzeichnet, dass** man die Destillation bei einer Verdampfertemperatur zwischen 60°C und 90°C unter einem Druck im Bereich von 700 mbar bis 10 mbar durchführt.

12. Verfahren nach einem der Ansprüche 9 und 10, **dadurch gekennzeichnet, dass** man das in der wässrigen Phase oder im Destillationsrückstand vorliegende Alkalimetallsalz der Trifluormethansulfinsäure gewinnt, indem man die folgenden Arbeitsgänge durchführt:
- zweites Ansäuern der gewonnenen Phase derart, dass das Trifluormethansulfinsäuresalz in seiner Säureform freigesetzt wird,
- Behandeln der abgetrennten wässrigen Phase, die im Wesentlichen die Trifluormethansulfinsäure umfasst, mit einem organischen Lösungsmittel, das zur Extraktion der Trifluormethansulfinsäure befähigt ist,
- Trennen der organischen Phase und der wässrigen Phase,
- Behandeln der organischen Phase mit einer basischen wässrigen Lösung derart, dass das Trifluormethansulfinsäuresalz in der wässrigen Phase gewonnen wird,
- Abdestillieren des Wassers und Ersetzen durch ein organisches Lösungsmittel zum Erhalt des Trifluormethansulfinsäuresalzes in organischer Lösung,
- Ausfällen des Trifluormethansulfinsäuresalzes mit einem Nichtlösungsmittel oder durch Kristallisation,
- Abtrennen und Gewinnen des Salzes.

13. Verfahren nach Anspruch 12, **dadurch gekennzeichnet, dass** man die gewonnene wässrige Phase einer zweiten Säurebehandlung mit einer starken Säure in einer solchen Menge, dass das Verhältnis zwischen der Zahl der Mole Säure, ausgedrückt als H⁺-Ionen, und der Zahl der Mole des Trifluormethansulfinsäuresalzes zwischen 1 und 10, vorzugsweise zwischen 2 und 4, variiert, unterwirft.

14. Verfahren nach Anspruch 12, **dadurch gekennzeichnet, dass** man die Trifluormethansulfinsäure mit einem organischen Lösungsmittel, vorzugsweise Methyl-tert.-butylether, Diisopropylether, Veratrol, extrahiert.

15. Verfahren nach Anspruch 12, **dadurch gekennzeichnet, dass** man am Ende der Extraktion zwei Phasen erhält: eine wässrige Phase, die die nach dem Ansäuern gebildeten Salze umfasst, und eine organische Phase, die das organische Lösungsmittel, die Trifluormethansulfinsäure und die restliche starke Säure umfasst.

16. Verfahren nach Anspruch 12, **dadurch gekennzeichnet, dass** man die gewonnene organische Phase einer Basenbehandlung zur Versalzung der erhaltenen Trifluormethansulfinsäure unterwirft, wobei die zugesetzte Basenmenge derart beschaffen ist, dass man einen pH-Wert zwischen 5 und 9 erhält.

17. Verfahren nach Anspruch 16, **dadurch gekennzeichnet, dass** man am Ende der Basenbehandlung zwei Phasen erhält: eine wässrige Phase, die das Trifluormethansulfinsäuresalz und das nach dem Ansäuern gebildete Salz umfasst, und eine organische Phase, die das organische Lösungsmittel umfasst.

18. Verfahren nach Anspruch 12, **dadurch gekennzeichnet, dass** man die abgetrennte wässrige Phase einer Destillation bei einer Verdampfertemperatur zwischen 60°C und 90°C unter einem Druck zwischen 1 mbar und 600 mbar unterwirft.

19. Verfahren nach Anspruch 12, **dadurch gekennzeichnet, dass** man den Destillationsrückstand mit einem organischen Lösungsmittel vom Alkohol-, Keton- oder Ester-Typ, vorzugsweise Isopropanol, behandelt.

20. Verfahren nach Anspruch 12, **dadurch gekennzeichnet, dass** man die Trifluormethansulfinsäure durch Behandlung der erhaltenen Phase mit einem Nichtlösungsmittel, vorzugsweise einem aliphatischen, cycloaliphatischen oder aromatischen Kohlenwasserstoff oder einem aliphatischen oder aromatischen Kohlenwasserstoff, ausfällt.

21. Verfahren nach Anspruch 12, **dadurch gekennzeichnet, dass** man das Trifluormethansulfinsäuresalz durch Filtrieren abtrennt und eine organische Phase, die das Lösungsmittel vom Alkohol-Typ und das Nichtlösungsmittel umfasst, gewinnt.

22. Verfahren nach Anspruch 1, **dadurch gekennzeichnet, dass** man das in der wässrigen Phase vorliegende Alkalimetallsalz der Trifluormethansulfinsäure gewinnt, indem man die wässrige Phase den folgenden Schritten unterwirft:
- Aufkonzentrieren der abgetrennten wässrigen Phase,
- Behandeln der wässrigen Phase, die im Wesentlichen das Trifluormethansulfinsäuresalz, die nach dem Ansäuern gebildeten Salze und die den Salzverunreinigungen entsprechenden Säureformen umfasst, mit einem organischen Lösungsmittel, das zur Extraktion des Trifluormethansulfinsäuresalzes befähigt ist,
- Abtrennen der suspendierten Salze,
- Trennen der organischen Phase und der wässrigen Phase,
- Gewinnen des Trifluormethansulfinsäuresalzes aus der abgetrennten organischen Phase.

23. Verfahren nach Anspruch 22, **dadurch gekennzeichnet, dass** man das Reaktionsmedium zur Erhöhung der Konzentration des Trifluormethansulfinsäuresalzes derart aufkonzentriert, dass in der erhaltenen konzentrierten Lösung zwischen 2 und 30%, vorzugsweise zwischen 5 und 20%, Wasser vorliegen.

24. Verfahren nach Anspruch 23, **dadurch gekennzeichnet, dass** man Wasser durch Destillation unter Normaldruck oder einem darunter liegenden Druck oder durch Einleiten eines Dampf- oder Inertgasfluids entfernt.

25. Verfahren nach Anspruch 22, **dadurch gekennzeichnet, dass** man das Trifluormethansulfinsäuresalz mit einem organischen Lösungsmittel vom Alkohol-, Keton- oder Ester-Typ extrahiert.

26. Verfahren nach Anspruch 25, **dadurch gekennzeichnet, dass** man am Ende der Extraktion eine organische Phase, die im Wesentlichen das Trifluormethansulfinsäuresalz umfasst, und eine wässrige Phase, die die nach dem Ansäuern gebildeten Salze und die den Salzverunreinigungen entsprechenden Säureformen umfasst, erhält.

27. Verfahren nach Anspruch, 26, **dadurch gekennzeichnet, dass** man die wässrige Phase und die
organische Phase trennt und die organische Phase durch Abdampfen des organischen Lösungsmittels derart aufkonzentriert, dass das Trifluormethansulfinsäuresalz ausfällt.

28. Verfahren nach Anspruch 1, **dadurch gekennzeichnet, dass** man das in der wässrigen Phase vorliegende Alkalimetallsalz der Trifluormethansulfinsäure gewinnt, indem man die wässrige Phase den folgenden Arbeitsgängen unterwirft:
- Eliminieren des in der wässrigen Phase, die das Alkalimetallsalz der Trifluormethansulfinsäure umfasst, vorliegenden Wassers,
- Zugeben eines organischen Lösungsmittels zum Auflösen des Trifluormethansulfinsäuresalzes,
- Gewinnen des Trifluormethansulfinsäuresalzes aus der abgetrennten organischen Phase.

29. Verfahren nach Anspruch 28, **dadurch gekennzeichnet, dass** man das Wasser durch azeotrope Destillation in Gegenwart eines Lösungsmittels, vorzugsweise vom Alkohol-Typ, eliminiert.

30. Verfahren nach Anspruch 28, **dadurch gekennzeichnet, dass** man das Trifluormethansulfinsäuresalz durch Abkühlen kristallisiert und dann gemäß herkömmlichen Techniken zur Feststoff/Flüssigkeit-Trennung, vorzugsweise durch Filtrieren, abtrennt.

31. Verfahren nach einem der Ansprüche 1 bis 30, **dadurch gekennzeichnet, dass** man eine Trocknung bei einer Temperatur zwischen 20°C und 80°C, vorzugsweise zwischen 30°C und 50°C, unter einem vorzugsweise verminderten Druck zwischen 0,1 und 200 mbar und vorzugsweise unter kontrollierter Inertgasatmosphäre, weiter bevorzugt Stickstoff oder Argon, durchführt.

32. Verfahren nach einem der Ansprüche 1 bis 31, **dadurch gekennzeichnet, dass** das erhaltene Trifluormethansulfinsäuresalz eine Reinheit größer gleich 90%, vorzugsweise größer gleich 95% und bevorzugt zwischen 95 und 98%, aufweist.
